(19) Europäisches Patentamt
European Patent Office
Office européen des brevets

(11) **EP 3 949 929 A1**

(12) **EUROPEAN PATENT APPLICATION**
published in accordance with Art. 153(4) EPC

(43) Date of publication:
**09.02.2022 Bulletin 2022/06**

(21) Application number: **20776655.1**

(22) Date of filing: **26.03.2020**

(51) International Patent Classification (IPC):
**A61H 5/00** (2006.01)        **A61B 3/024** (2006.01)
**A61B 3/08** (2006.01)

(52) Cooperative Patent Classification (CPC):
**A61B 3/024; A61B 3/08; A61H 5/00**

(86) International application number:
**PCT/JP2020/013565**

(87) International publication number:
**WO 2020/196715 (01.10.2020 Gazette 2020/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.03.2019 JP 2019062059**

(71) Applicant: **WASEDA UNIVERSITY
Shinjuku-ku
Tokyo 169-8050 (JP)**

(72) Inventors:
• **IWATA, Hiroyasu
Tokyo 169-8050 (JP)**
• **KATO, Ryoichi
Tokyo 169-8050 (JP)**
• **YASUDA, Kazuhiro
Tokyo 169-8050 (JP)**

(74) Representative: **Grünecker Patent- und
Rechtsanwälte
PartG mbB
Leopoldstraße 4
80802 München (DE)**

(54) **VISUAL PERCEPTION FUNCTION EVALUATION SYSTEM**

(57)    A visual perception function evaluation system 10 includes: a display device 12 configured to three-dimensionally display a three-dimensional test image 20 including an object 22 to a subject; a processing device 13 connected to the display device 12; and an input device 11 through which a reply related to whether the object 22 can be perceived is input from the subject to the processing device 13. The processing device 13 includes a display control means 15 for controlling the state of display of the test image 20 on the display device 12, and a neglect region specifying means 16 for specifying a neglect region in a three-dimensional space based on the reply. The display control means 15 controls display so that the three-dimensional position of a display point P varies over time. The neglect region specifying means 16 determines three-dimensional position information on a boundary part between a perception region and the neglect region based on a position of the display point P where it is replied that the object 22 cannot be perceived and an adjacent position of the display point P where it is replied that the object 22 can be perceived.

F I G . 1

## Description

Technical Field

The present invention relates to a visual perception function evaluation system, and particularly relates to a visual perception function evaluation system that can quantitatively evaluate a neglect region in a stereoscopic space, the neglect region being a region in which a subject having impaired visuospatial ability, such as a patient with unilateral spatial neglect, cannot perceive an object existing in front of the subject.

Background Art

Examples of higher brain dysfunction attributable to cerebrovascular disease include impaired visuospatial ability called unilateral spatial neglect with which a stimulus existing on a side opposite to a lesion in a brain is neglected. For example, a patient of unilateral spatial neglect due to damage on the right brain neglects a target existing in a left space in the visual space of the patient, and suffers troubles such as collision with the left side of a door when passing through the door, incapability of reading the left side of a printed document, and incapability of noticing dishes on the left side when eating. It is thought that unilateral spatial neglect symptoms include inattention in a proximal space near the patient (proximal spatial inattention), inattention in a distal space far from the patient (distal spatial inattention), and inattention in both spaces.

Various kinds of rehabilitation are performed on a patient with unilateral spatial neglect to extend the visual field of the patient, and the state of inattention of the patient needs to be identified in the rehabilitation. Thus, a test called behavioral inattention test (BIT) has been conventionally employed as a test for identifying the state of unilateral spatial neglect. In the BIT, a test sheet on which various line figures are illustrated is placed in front of a subject, and the state of unilateral spatial neglect of the subject is evaluated based on a reply of a part that can be visually perceived by the subject. However, the BIT is an on-desk test using a sheet, can perform only planar and limited evaluation of proximal spatial inattention at a constant distance from the subject, and cannot perform inattention evaluation in the entire stereoscopic space including the distal space. Accordingly, with the BIT, it is impossible to clearly determine the range of a neglect region in which perception is impossible in a visual space in front of the subject. Thus, rehabilitation needs to be intuitively performed on the patient, and it is impossible to effectively perform rehabilitation for decreasing the neglect region. Furthermore, according to medical knowledge such as neuropsychological knowledge, different responsible regions at the brain center correspond to the proximal space and the distal space, and thus ignoring evaluation specialized for each of the proximal space and the distal space is needed.

Patent Literature 1 discloses a visual perception test system that examines the degree and contents of impaired visuospatial ability such as unilateral spatial neglect. In the system, a stereoscopic image is presented to a subject and the position of the stereoscopic image is input, and accordingly, the degree of disorder of the subject is detected based on comparison with results of a healthy subject.

Citation List

Patent Literature

Patent Literature 1: Japanese Patent Laid-Open No. 2011-212430

Summary of Invention

Technical Problem

However, the system of Patent Literature 1 is based on a premise that a subject perceives the existence of a stereoscopic image, and is configured to calculate the distance between the stereoscopic image and the center between the eyes of the subject at the perception and determine the degree of disorder of the patient in accordance with the distance, but does not stereoscopically specify the neglect region. Thus, with the system of Patent Literature 1, it is impossible to specifically identify a spatial neglect situation such as the spatial range of the neglect region existing in the visual space of the subject, and thus it is impossible to perform effective rehabilitation in accordance with the situation identification.

The present invention is achieved with focus on such an inconvenience and has an objective to provide a visual perception function evaluation system that can three-dimensionally quantitatively identify an neglect region in the visual space of a subject.

Solution to Problem

To achieve the objective, the present invention is a visual perception function evaluation system for three-dimensionally identifying mainly a neglect region and a perception region and evaluating a neglect symptom of a subject having impaired visuospatial ability, the neglect region being a region in which the subject cannot perceive an object in a visual space, the perception region being a region in which the subject can perceive the object. The visual perception function evaluation system includes: a display device configured to display a three-dimensional test image including a predetermined object so that the subject can have a stereoscopic view; a processing device connected to the display device and configured to perform predetermined processing; and an input device through which a reply related to whether the object can be perceived is input from the subject to the processing device. The processing device includes a display control means for controlling the state of display of the test image on the display device, and a neglect region specifying means for specifying the neglect region in a three-dimensional space in front of the subject based on the reply. The display control means causes the display device to display the object so that the three-dimensional position of a display point of the object in the stereoscopic view of the subject varies over time. The neglect region specifying means determines three-dimensional position information on a boundary part between the perception region and the neglect region based on a position of the display point where it is replied that the object cannot be perceived and an adjacent position of the display point where it is replied that the object can be perceived.

Advantageous Effects of Invention

According to the present invention, since the three-dimensional position of the boundary part between the perception region and the neglect region is specified by the neglect region specifying means, it is possible to three-dimensionally quantitatively identify the existence of the neglect region in the visual space of the subject. Thus, it is possible to effectively provide different rehabilitation such as rehabilitation corresponding to proximal spatial inattention, distal spatial inattention, or the like to the subject, depending on the situation of existence of a neglect space in the visual space. In addition, it is possible to digitize data on three-dimensional positions in the range of the neglect region and quantitatively identify a medical treatment effect through rehabilitation by comparing the situation of existence of the neglect region before and after the rehabilitation. Moreover, it is possible to three-dimensionally visualize the neglect region and present the neglect space of a patient as the subject in an easily understandable manner to the patient, a therapist in charge of rehabilitation, or the like. Furthermore, position information on the neglect region is automatically specified, and thus it is possible to transfer data to another medical treatment support system.

Brief Description of Drawings

[Figure 1] Figure 1 is a block diagram of the configuration of a visual perception function evaluation system according to the present embodiment.
[Figure 2] Figure 2 (A) to (C) are diagrams illustrating test images for description of display change of an object over time.
[Figure 3] Figure 3 (A) is a conceptual diagram illustrating a space coordinate, and Figure 3 (B) is a conceptual diagram illustrating the space coordinate when viewed at an angle different from that for (A).
[Figure 4] Figure 4 is a conceptual diagram illustrating a plane coordinate.

[Figure 5] Figure 5 (A) is a diagram for description of setting of boundary points of a neglect region in the left region on the plane coordinate, and Figure 5 (B) is a diagram for description of setting of boundary points of a neglect region in the right region on the plane coordinate.
[Figure 6] Figure 6 (A) to (C) are diagrams illustrating exemplary display of the neglect region for three plane coordinates on the same subject.
[Figure 7] Figure 7 is a diagram for description of a perception angle.

Description of Embodiment

An embodiment of the present invention will be described below with reference to the accompanying drawings.

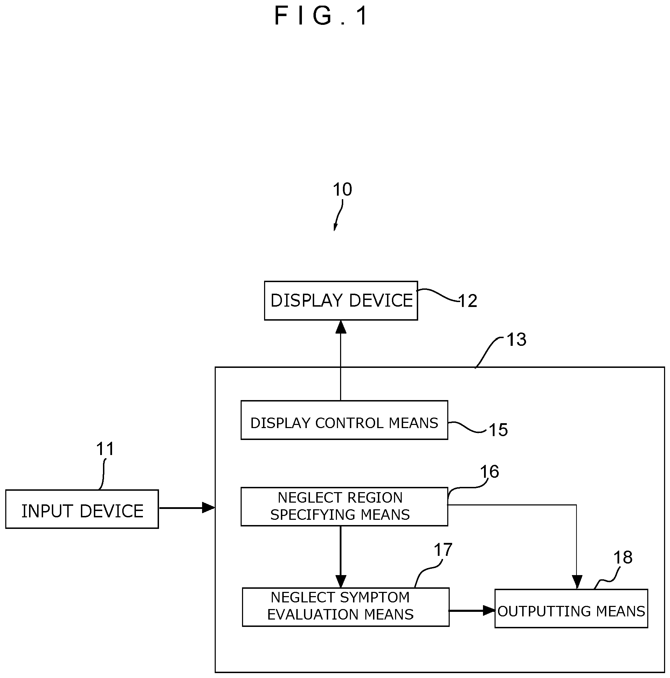

Figure 1 is a block diagram illustrating the configuration of a visual perception function evaluation system according to the present embodiment. In this drawing, this visual perception function evaluation system 10 is a system for quantitatively evaluating the state of impaired visuospatial ability of a subject, and in the present embodiment, is a system that allows a patient having a unilateral spatial neglect symptom to three-dimensionally identify, in the visual space of the patient, a perception region in which an object can be perceived and a neglect region in which the object cannot perceived.
The visual perception function evaluation system 10 includes an input device 11 through which an inputter such as a patient as the subject or a therapist accompanying the patient inputs various kinds of information into the system, a display device 12 capable of stereoscopically presenting a three-dimensional test image including a predetermined object to the subject, and a processing device 13 connected to the display device 12 and configured to perform predetermined processing.
Various kinds of information including a reply of whether the object in the test image presented to the subject by the display device 12 is visually perceived by the subject is input to the input device 11, and the input information is then transmitted to the processing device 13. Note that, various input instruments, such as a button, a pedal, a keyboard, a touch panel, a mouse, with which information can be input by a body operation of the inputter, and various kinds of input devices including a line-of-sight input device through which motion of the line of sight of the subject or the like is traced by a camera and information is input in accordance with the motion may be employed as the input device 11 as long as various kinds of information can be input and transmitted. When a line-of-sight input device is used, manual information input by the inputter is unnecessary and the perception region can be detected only by activating the system.
As the display device 12, a display device including a head-mounted display that is to be mounted on the head of the subject and having a well-known structure that allows the test image transferred from the processing device 13 to be presented such that the test image can be stereoscopically viewed in front of the subject. Note that, any other instrument may be employed as the display device 12 as long as the test image can be three-dimensionally presented to the subject.

The processing device 13 includes a computer including an arithmetic processing device such as a CPU and including a storage device such as a memory or a hard disk, and includes a computer program installed therein for causing the computer to function as each means described below.

The processing device 13 includes a display control means 15 for transmitting a test image stored in advance to the display device 12 and controlling the state of display of the test image on the display device 12, an neglect region specifying means 16 specifying, based on a reply input through the input device 11, the neglect region in which the subject cannot perceive the object in a three-dimensional space in front of the subject, a neglect symptom evaluation means 17 evaluating a neglect symptom of the subject based on the neglect region specified by the neglect region specifying means 16, and an outputting means 18 outputting data obtained by the neglect region specifying means 16 and the neglect symptom evaluation means 17 to an external device, instrument, and/or system.

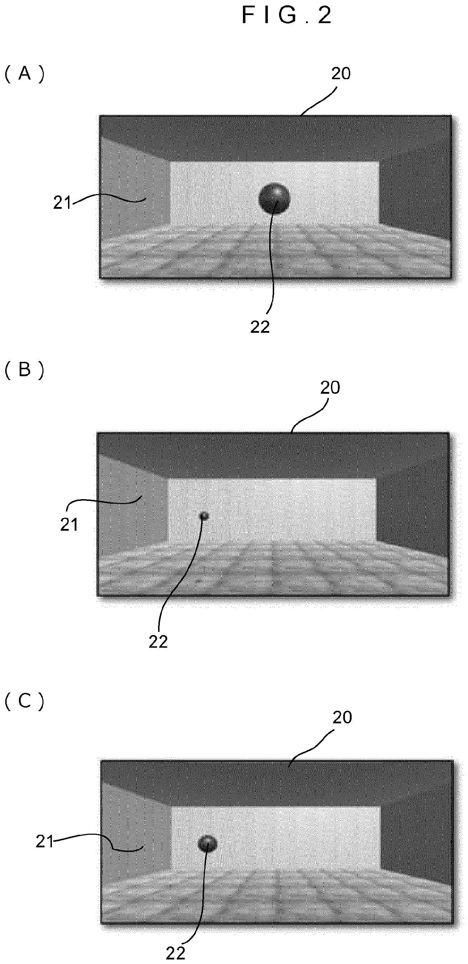

The display control means 15 transmits a test image 20, as exemplarily illustrated in Figure 2, to the display device 12 so that an immersive three-dimensional virtual reality space (hereinafter referred to as "VR space") set in advance can be visually recognized by the subject from a first-person viewpoint. The test image 20 is not particularly limited but is made of a predetermined background image 21 and a spherical object 22 that is superimposed on the background image 21 and moves in the background image 21.

As illustrated in (A) to (C) of the drawing, the object 22 is presented to the subject such that the three-dimensional position of the object 22 in a stereoscopic view of the subject through the display device 12 randomly changes relative to the background image 21 over time. Furthermore, the object 22 is randomly displayed, based on a presentation rule to be described later, at the position of any one of a plurality of display points set in advance in the VR space, and is displayed at a display point at another different position after a reply of whether the object 22 displayed at the position can be perceived. Note that, the appearance order of the object 22 is set to be random so as not to cause visual induction that would be an obstructive factor for accurate neglect region evaluation of the subject.

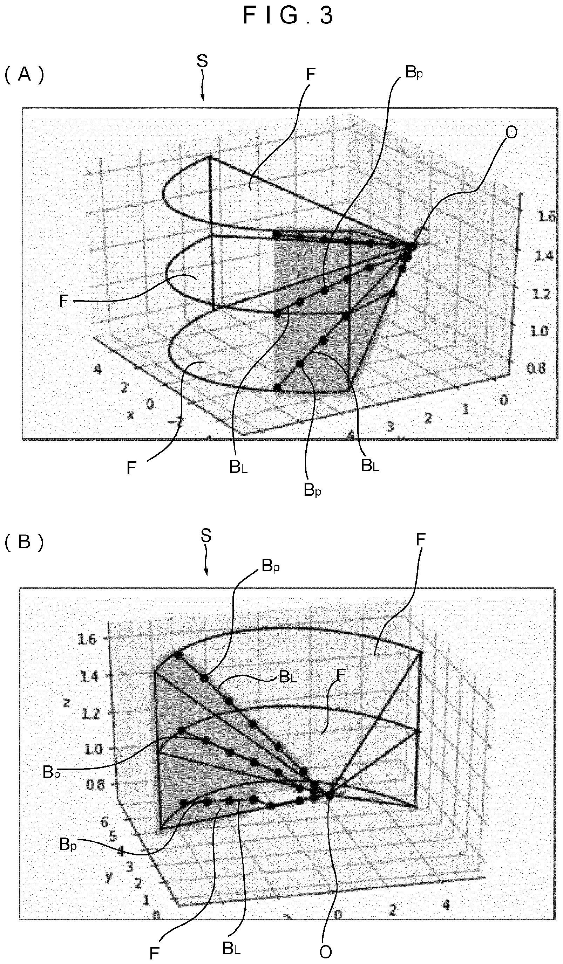

In the present embodiment, the object 22 is displayed at any of display points set in advance in a three-dimensional space coordinate S (refer to Figure 3) set in the VR space. The space coordinate S is a coordinate system with an origin O set to be an eyeball position at the center of the body trunk when the display device 12 is mounted on the subject, and is made of plane coordinates F disposed at different tilt angles from the origin O in the depth direction in the VR space. The plane coordinates F are not particularly limited but are set at three levels of height with the tilt angles of - 4°, 0°, and 4° relative to the eye line height of the subject.

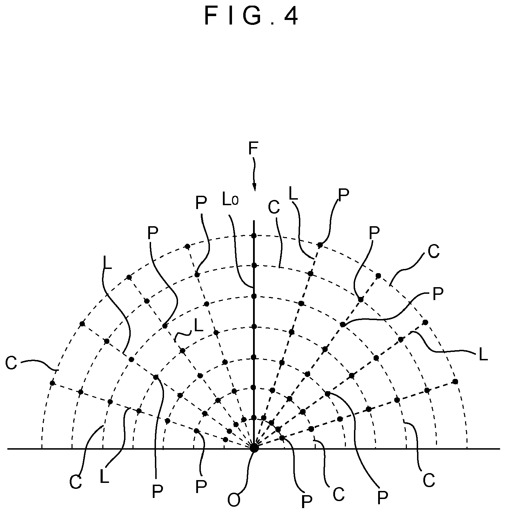

As illustrated in Figure 4, each plane coordinate F is made of a polar coordinate system constituted by the separation distance from the origin O and the rotation angle about the origin O. In the drawing, display points P are set at intersection points between a plurality of angle straight lines L radially extending from the origin O in the depth direction at predetermined rotation angles and distance circles C concentrically disposed about the origin O with mutually different predetermined separation distances as the radius. The angle straight lines L are symmetrically disposed at predetermined rotation angles (deviation angles) in right and left regions of a median position line $L_0$ extending straightforward from the center of the body. In the illustrated example, the angle straight lines L are provided at the interval of 18° up to the deviation angles of positive and negative 90°. The distance circles C are provided at constant interval and set in seven kinds with different distances (radii). In other words, the display points P are set at a plurality of separation distances with a constant interval therebetween and a plurality of respective deviation angles with a constant interval therebetween.

Note that, the space coordinate S is not limited to the configuration described above, but the configuration and disposition aspect of the plane coordinates F may be changed, or a coordinate system of another configuration such as a spherical polar coordinate may be employed.

The object 22 is displayed at a display point P at one predetermined place in the VR space. Then, whether the object 22 at the display point P is perceived by the subject is input to the input device 11, and thereafter the object 22 is displayed at a display point P at another position. The display order of the object 22 is at random among the display points P on the respective plane coordinates F as long as the presentation rule described below is obeyed, and display of the object 22 is continued for all separation distances from the origin O on each plane coordinate F until it is detected in which angle range the object 22 can be perceived by the subject in the lateral direction from the body center of the subject.

In the presentation rule, for example, the object 22 is initially randomly displayed at any display point P on the median position line $L_0$ on any plane coordinate F. Then, when it is replied that the display point P on the median position line $L_0$ in the plane coordinate F can be perceived through the input device 11, the object 22 is moved and displayed as follows. Specifically, the object 22 is displayed at an adjacent display point P existing on the left side of the subject at the same separation distance in the plane coordinate F. This display of the object 22 at the same separation distance is performed at each display point P positioned further on the left side until it is replied that perception is impossible.

When it is initially replied that the object 22 cannot be perceived at the display point P on the median position line $L_0$ in the plane coordinate F, the object 22 is displayed at an adjacent display point P existing on the right side of the subject at the same separation distance in the plane coordinate F. This display of the object 22 at the same separation distance is performed at each display point P positioned further on the right side until it is replied that perception is possible.

The above-described presentation rule is an algorithm in which, since unilateral spatial neglect often occurs in a space on the left side of the body central line due to characteristics of the neglect, the initial appearance position of the object 22 is set as the deviation angle of 0° and the next appearance position is shifted to a neglect side (the left side) or a non-neglect side (the right side) in accordance with a reply at each separation distance. Accordingly, it is possible to efficiently specify the neglect region for the subject such as a stroke patient having significantly decreased physical strength, thereby reducing the time of the above-described measurement for the specification.

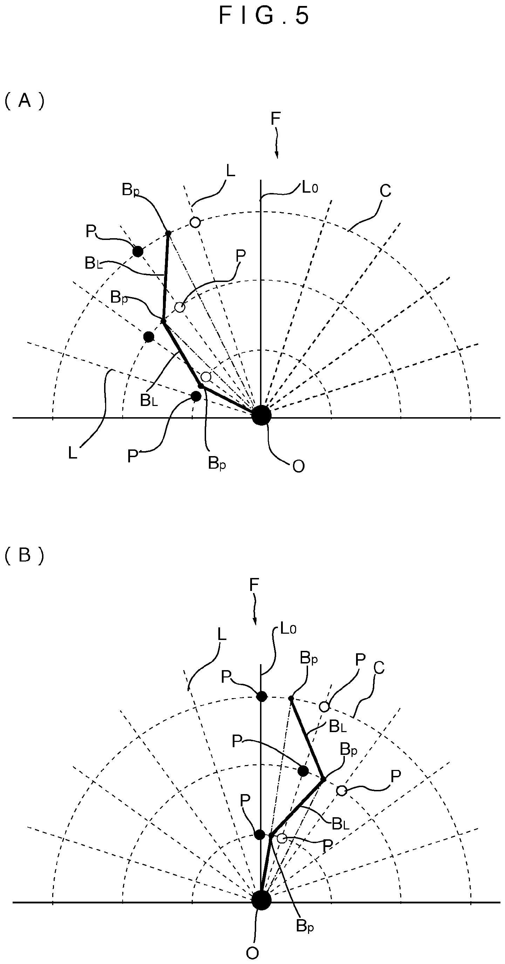

The neglect region specifying means 16 determines, at a height h specified for each plane coordinate F and at each separation distance r on the plane coordinate F, three-dimensional position information on a boundary part between the perception region and the neglect region based on three-dimensional position information on a display point P at which the object 22 is last displayed. Specifically, as illustrated in Figure 5, a boundary point $B_P$ of whether perception is possible at the separation distance r is determined to be a point existing at an angle in the middle between a display point P at which the object 22 is last displayed and an adjacent display point P previously displayed at the same separation distance r in the same plane coordinate F. More specifically, as illustrated in Figure 5 (A), the boundary point $B_P$ in the left region on the plane coordinate F is determined to be a position between a display point P (black circle in the drawing) where it is initially replied that perception is impossible and an adjacent display point P (white circle in the drawing) where it is previously replied that perception is possible at the same separation distance r. In addition, as illustrated in (B) of the drawing, the boundary point $B_P$ in the right region on the plane coordinate F is determined to be a position between a display point P (white circle in the drawing) where it is initially replied that perception is possible and an adjacent display point P (black circle in the drawing) where it is previously replied that perception is impossible at the same separation distance r.

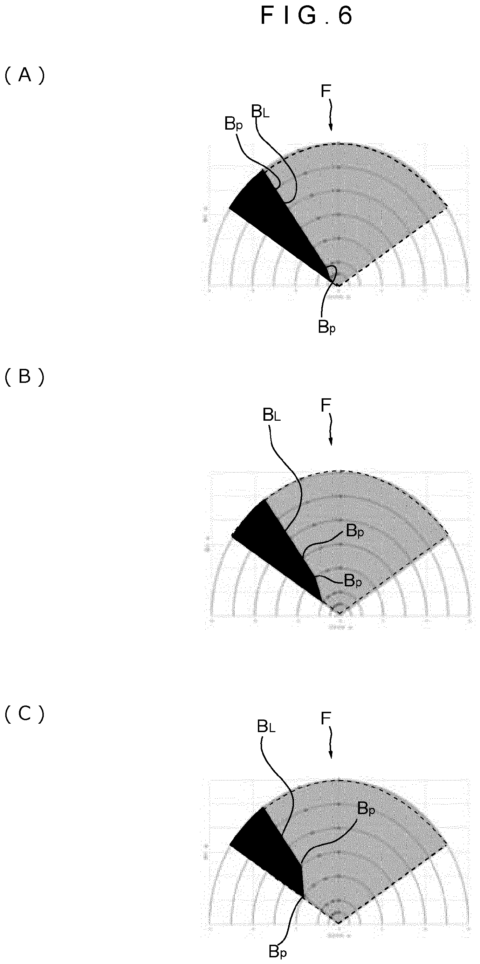

The boundary point $B_P$ is specified at each height h and each separation distance r in the VR space, and a boundary line $B_L$ between the perception region and the neglect region is determined to be a line connecting the boundary points $B_P$ vertically adjacent to each other in Figure 5 on each plane coordinate F. In addition, the boundary surface between a perception space as the perception region and a neglect space as the neglect region is determined to be a surface including the boundary line $B_L$ specified on each plane coordinate F. As a result, a position range of the neglect region is specified in the three-dimensional space, and various kinds of data based on the position range can be output from the outputting means 18. For example, position data on the neglect space can be provided to a non-illustrated rehabilitation instrument or system based on the output data. Moreover, as illustrated in Figure 6 (A) to (C), another display device may be used to perform graphic display of the neglect region (black part in the drawing) for each of three coordinate planes F on the same subject, and as illustrated in Figure 3, another display device may be used to perform display of the neglect space (thick color part in the drawing) by projection mapping or the like.

The neglect symptom evaluation means 17 calculates, based on data on the position in the neglect region specified by the neglect region specifying means 16, an indicator value for quantitatively evaluating the state of perception in the visual space of the subject.

The indicator value is not particularly limited, but may be, for example, a perception angle indicating an angle range in which it is possible to perceive an object existing in a visual space in which visual perception of a human is effective, an neglect angle obtained by subtracting the perception angle of a subject from the perception angle of a healthy subject set in advance, the area of the neglect region on each plane coordinate F, and/or the volume of the neglect space including the neglect region on each plane coordinate F.

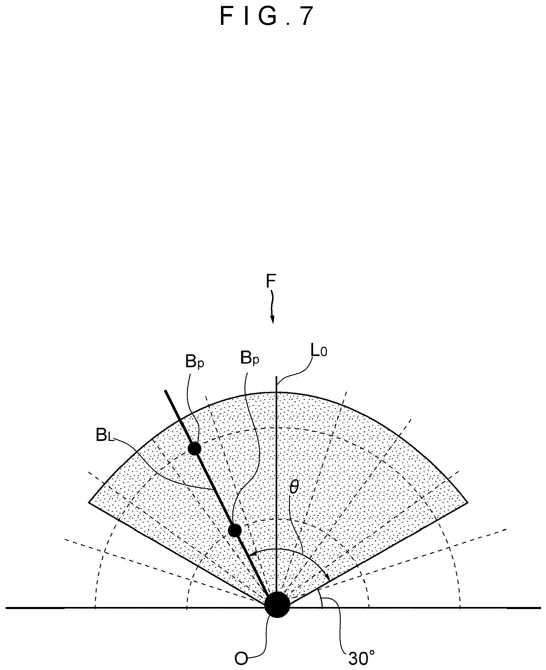

The perception angle and the neglect angle are calculated for each boundary point $B_P$ on each plane coordinate F. The perception angle (view angle) of a healthy subject is stored as 120° in advance. Since the degree of perception in the left space of a subject is low due to characteristics of unilateral spatial neglect as described above, the perception angle is defined as follows to indicate a perception range from the right space. Specifically, as illustrated in Figure 7, a perception angle θ is defined to be the angle between a boundary line on the right side in the perception range (hatched part in the drawing) of a healthy subject on the plane coordinate F, in other words, a straight line rotated by 30° from the horizontal axis on the plane coordinate F when the perception angle of a healthy subject is defined to be 120° and a straight line connecting the origin O and the boundary point $B_P$.

In addition, a perception ratio as an indicator related to the perception function of the subject in comparison with a healthy subject, and a search ratio as an indicator related to the motor function of the subject in comparison with a healthy subject are calculated as the indicator value for each boundary point $B_P$ on each plane coordinate F.

The perception ratio is a value calculated from the coordinates of each boundary point $B_P$ obtained as a result of a reply of whether the object 22 can be perceived in a head fixed state in which the boundary point $B_P$ is measured through the above-described procedure under a condition that the head of the subject is fixed, and is the ratio of the perception angle θ of the subject relative to the perception angle (120°) of a healthy subject.

The search ratio is a value calculated by additionally using the coordinates of each boundary point $B_P$ obtained as a result of a reply of whether the object 22 can be perceived in a head unfixed state in which the boundary point $B_P$ is measured through the above-described procedure under a condition that the head of the subject can be moved. As described later, the search ratio is a ratio between a requested search angle corresponding to request search performance indicating search motion capability requested for the subject in accordance with the degree of perception and a motion perception angle corresponding to search performance indicating the actual search motion capability of the subject.

The requested search angle indicates what turn angle of turn motion is needed for the perception function of the subject with the perception angle θ in the head fixed state to ensure a perception range equivalent to that of a healthy subject, in other words, enable space perception in the angle range of 180° from the front side of the head to the right and left sides. For example, the needed turn angle of a healthy subject is 30° to the right and left since the perception angle thereof is 120° as described above. Thus, when $θ_{hr}$ represents the perception angle at the height h and the separation distance r in the head fixed state, a requested search angle $g(θ_{hr})$ is expressed by an expression below based on the above-described definition of the perception angle.

$$g(θ_{hr}) = 150 - θ_{hr}$$

The motion perception angle is an angle difference $φ_{hr}$ of the perception angle calculated at each height h and each separation distance r between the head fixed state and the head unfixed state, and the search ratio at the height h and the separation distance r is given by $φ_{hr}/g(θ_{hr})$.

In this manner, it is possible to easily estimate which of the perception function and motor function of a patient has a defect by determining the perception ratio and the search ratio. The motor function and the requested search angle needed for the patient differ depending on the degree of the perception function of the patient. Thus, how much the perception region is covered is unknown only with simple verification of patient motion, but evaluation using the perception ratio and the search ratio clarifies whether to reinforce the perception function or the motor function, thereby allowing effective rehabilitation specialized for this reinforcement point.

Note that, with the perception ratio and/or the search ratio, it is possible to perform scoring of overall neglect evaluation, such as calculation of a synthesized indicator value of the perception ratio and the search ratio by, for example, multiplying the ratios for each height h and/or each separation distance r or multiplying all values of the ratios.

In the embodiment, the visual perception function evaluation system 10 is used to evaluate the visual perception of a patient with unilateral spatial neglect, but the present invention is not limited thereto, and the visual perception function evaluation system 10 may be used to evaluate the visual perception of a patient having any other similar perception disorder.

Moreover, the configuration of device components in the present invention is not limited to the illustrated exemplary configuration, but the configuration may be modified in various kinds of manners as long as substantially same effects are achieved.

Reference Signs List

10  visual perception function evaluation system
11  input device
12  display device
13  processing device
15  display control means
16  neglect region specifying means
17  neglect symptom evaluation means
20  test image
22  object
P   display point
$B_P$  boundary point

**Claims**

1. A visual perception function evaluation system for three-dimensionally identifying a neglect region and a perception region and evaluating a neglect symptom of a subject having impaired visuospatial ability, the neglect region being a region in which the subject cannot perceive an object in a visual space, the perception region being a region in which the subject can perceive the object, the visual perception function evaluation system comprising:

   a display device configured to display a three-dimensional test image including a predetermined object so that the subject can have a stereoscopic view;
   a processing device connected to the display device and configured to perform predetermined processing; and
   an input device through which a reply related to whether the object can be perceived by the subject is input to the processing device, wherein

the processing device includes a display control means for controlling the state of display of the test image on the display device, and a neglect region specifying means for specifying the neglect region in a three-dimensional space in front of the subject based on the reply,

the display control means causes the display device to display the object so that the three-dimensional position of a display point of the object in the stereoscopic view of the subject varies over time, and

the neglect region specifying means determines three-dimensional position information on a boundary part between the perception region and the neglect region based on a position of the display point where it is replied that the object cannot be perceived and an adjacent position of the display point where it is replied that the object can be perceived.

2. The visual perception function evaluation system according to claim 1, wherein the display control means gradually moves the object from a central position in front of the subject toward a side position in a range in which the height in front of the subject and the separation distance from the subject are constant until the reply that perception is impossible is obtained.

3. The visual perception function evaluation system according to claim 2, wherein the display control means initially displays the object at the display point at the central position in the range in which the height and the separation distance are constant, and when it is replied that perception is impossible, the display control means moves the object to the right of the subject until the reply that perception is possible is obtained.

4. The visual perception function evaluation system according to claim 2, wherein the display control means randomly displays the object at the display points between which the height and the separation distance are different.

5. The visual perception function evaluation system according to claim 1, wherein the neglect region specifying means determines, in a range in which the height in front of the subject and the separation distance from the subject are constant, a boundary point of the neglect region to be between the position of the display point where it is replied that perception is impossible and the adjacent position of the display point where it is replied that perception is possible.

6. The visual perception function evaluation system according to claim 1, further comprising a neglect symptom evaluation means for calculating, based on the neglect region, an indicator value for quantitatively evaluating the state of perception in the visual space, wherein the neglect symptom evaluation means calculates, as the indicator value, a perception angle indicating an angle range in which the object existing in the visual space can be perceived, a neglect angle obtained by subtracting the perception angle of the subject from the perception angle of a healthy subject, which is set in advance, the area of the neglect region on a predetermined plane in the visual space, and/or the volume of the neglect region.

7. The visual perception function evaluation system according to claim 1, further comprising a neglect symptom evaluation means for calculating, based on the neglect region, an indicator value for quantitatively evaluating the state of perception in the visual space, wherein

the neglect symptom evaluation means calculates, as the indicator value, a perception ratio that is an indicator related to a perception function of the subject, and a search ratio that is an indicator related to a motor function of the subject,

the perception ratio is the ratio of a perception angle of the subject relative to the perception angle of the healthy subject in a head fixed state in which the head of the subject is fixed, the perception angle indicating an angle range in which the object existing in the visual space can be perceived, and

the search ratio is a ratio between a requested search angle and a motion perception angle in a head unfixed state in which the head of the subject can be moved, the requested search angle being a turn angle requested with respect to the perception angle in the head fixed state for the subject to ensure a perception range equivalent to the perception range of the healthy subject, the motion perception angle being an angle difference of the perception angle between the head fixed state and the head unfixed state.

F I G . 1

10

DISPLAY DEVICE ⌐12

13

DISPLAY CONTROL MEANS ⌐15

11

INPUT DEVICE → NEGLECT REGION SPECIFYING MEANS ⌐16

17

NEGLECT SYMPTOM EVALUATION MEANS → OUTPUTTING MEANS

18

# F I G . 2

( A )

( B )

( C )

# F I G . 3

( A )

( B )

# FIG.4

# FIG. 5

(A)

(B)

# FIG.6

(A)

(B)

(C)

# F I G . 7

## INTERNATIONAL SEARCH REPORT

| International application No. |
|---|
| PCT/JP2020/013565 |

A. CLASSIFICATION OF SUBJECT MATTER
A61H 5/00(2006.01)i; A61B 3/024(2006.01)i; A61B 3/08(2006.01)i
FI: A61B3/024; A61B3/08; A61H5/00 Z

According to International Patent Classification (IPC) or to both national classification and IPC

B. FIELDS SEARCHED

Minimum documentation searched (classification system followed by classification symbols)
A61H5/00; A61B3/024; A61B3/08

Documentation searched other than minimum documentation to the extent that such documents are included in the fields searched
Published examined utility model applications of Japan       1922–1996
Published unexamined utility model applications of Japan     1971–2020
Registered utility model specifications of Japan             1996–2020
Published registered utility model applications of Japan     1994–2020

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2001-79050 A (JAPAN SCIENCE AND TECHNOLOGY CORP.) 27.03.2001 (2001-03-27) paragraph [0163] | 1-7 |
| A | JP 2011-212430 A (THE UNIVERSITY OF TOKYO) 27.10.2011 (2011-10-27) paragraphs [0033], [0034] | 1-7 |
| A | 藤村誠，井上一暉，藤井秋希良，東登志夫，半側空間無視症状の評価支援システムの一検討，電子情報通信学会技術研究報告，12 October 2017, vol. 117, no. 250, pp. 107-111, page 108, left column, lines 13-20, page 108, left column, lines 13-20, (FUJIMURA, Makoto, INOUE, Kazuki, FUJI, Akira, HIGASHI, Toshio, "Study of Assessment Support System of Unilateral Spatial Neglect", IEICE technical report) | 1-7 |

☒ Further documents are listed in the continuation of Box C.     ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 04 June 2020 (04.06.2020) | 16 June 2020 (16.06.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
|---|
| PCT/JP2020/013565 |

C (Continuation).   DOCUMENTS CONSIDERED TO BE RELEVANT

| Category* | Citation of document, with indication, where appropriate, of the relevant passages | Relevant to claim No. |
|---|---|---|
| A | US 2011/0004412 A1 (SHAHAF, Goded) 06.01.2011 (2011-01-06) paragraph [0116] | 1-7 |

Form PCT/ISA/210 (continuation of second sheet) (January 2015)

**INTERNATIONAL SEARCH REPORT**
Information on patent family members

International application No.

PCT/JP2020/013565

| Patent Documents referred in the Report | Publication Date | Patent Family | Publication Date |
|---|---|---|---|
| JP 2001-79050 A | 27 Mar. 2001 | (Family: none) | |
| JP 2011-212430 A | 27 Oct. 2011 | (Family: none) | |
| US 2011/0004412 A1 | 06 Jan. 2011 | WO 2009/069136 A2 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011212430 A